# FASCICULE DE BREVET EUROPEEN

(11) **EP 2 521 533 B1**
(45) Date de publication et mention de la délivrance du brevet: **02.05.2018**
(21) Numéro de dépôt: 10801666.8
(22) Date de dépôt: 13.12.2010
(51) Int. Cl.: A61Q 1/06, A61K 8/92

(54) **COMPOSITION COSMÉTIQUE, COMPOSÉ ET PROCÉDÉ DE TRAITEMENT COSMÉTIQUE**
KOSMETISCHE ZUSAMMENSETZUNG, KOSMETISCHE BEHANDLUNG, VERBINDUNG
COSMETIC COMPOSITION, COSMETIC TREATMENT METHOD AND COMPOUND

(30) Priorité: 02.02.2010 US 300523 P; 04.01.2010 FR 1050012
(43) Date de publication de la demande: 14.11.2012
(73) Titulaire: L'Oréal, 75008 Paris (FR)
(72) Inventeur: CHODOROWSKI-KIMMES, Sandrine, F-60300 Senlis (FR); JAGER LEZER, Nathalie, F-91370 Verrieres-le-Buisson (FR)
(74) Mandataire: Kromer, Christophe
(86) Numéro de dépôt international: PCT/FR2010/052695
(87) Numéro de publication internationale: WO 2011/080448

(56) Documents cités:
- EP-A1- 2 140 858
- WO-A1-2011/147696
- FR-A1- 2 825 628
- US-A1- 2006 018 856
- US-A1- 2007 093 639
- Patricia Y. W. Dankers, Martin C. Harmsen, Linda A. Brouwer, Marja J. A. Van Luyn & E. W. Meijer: "A modular and supramolecular approach to bioactive scaffolds for tissue engineering", Nature Materials, vol. 4, no. 7 juillet 2005 (2005-07), pages 568-574, XP002606540, DOI: 10.1038/nmat1418 Extrait de l'Internet: URL:http://www.nature.com/nmat/journal/v4/ n7/pdf/nmat1418.pdf [extrait le 2010-10-18]
- D. J. M. van Beek, Martijn A. J. Gillissen, Bart A. C. van As, Anja R. A. Palmans, and Rint P. Sijbesma: "Supramolecular Copolyesters with Tunable Properties", Macromolecules, vol. 40, no. 17 31 juillet 2007 (2007-07-31), pages 6340-6348, XP002606541, DOI: 10.1021/ma0705927 Extrait de l'Internet: URL:http://pubs.acs.org/doi/pdf/10.1021/ma 0705927 [extrait le 2010-10-21]
- FOLMER B J B ET AL: "SUPRAMOLECULAR POLYMER MATERIALS: CHAIN EXTENSION OF TELECHELIC POLYMERS USING A REACTIVE HYDROGEN-BONDING SYNTHON", ADVANCED MATERIALS, WILEY VCH VERLAG, DE LNKD- DOI:10.1002/1521-4095(200006)12:12<874::AI D-ADMA874>3.3.CO;2-3, vol. 12, no. 12, 16 juin 2000 (2000-06-16) , pages 874-878, XP000959548, ISSN: 0935-9648

## Description

La présente invention concerne une composition cosmétique comprenant un composé obtenu par réaction entre une cire fonctionnalisée et un groupe de jonction, ainsi qu'un procédé de traitement cosmétique employant ladite composition, et lesdits composés.
Dans de nombreuses compositions cosmétiques, on utilise des cires en mélange avec des huiles et d'autres corps gras, afin de donner de la consistance aux compositions. Ces compositions trouvent des applications principalement dans le domaine du maquillage et/ou du soin de la peau et/ou du cheveu. Les cires sont couramment utilisées pour apporter de la texture aux compositions; elles ont généralement une grande stabilité dans le temps.
Toutefois, des problèmes de stabilité mécanique et/ou de compatibilité entre les cires et les produits volatils, notamment les huiles volatiles généralement présentes dans les compositions cosmétiques peuvent se poser. On constate en effet, pour une faible teneur en cires, une dureté insuffisante de la composition, stick par exemple, qui peut être à l'origine de problèmes de stabilité ou d'utilisation. La simple augmentation de la proportion de cires durcissantes ne permet pas de résoudre ces problèmes car elle se traduit généralement par une dégradation des propriétés cosmétiques du produit qui devient inconfortable à porter.
En effet, dans certains cas, les compositions peuvent présenter une sensation de collant, de dépôt gras et peuvent être difficiles à appliquer, notamment lorsqu'elles comprennent de trop grandes quantités de cires. De plus, le dépôt formé sur le support kératinique peut être inhomogène surtout à haute concentration en cire.
Un des objectifs de la présente invention est de proposer des cires permettant d'obtenir un durcissement adéquat, c'est-à-dire ayant un pouvoir de gélification adéquat, de manière à limiter la quantité de cires dans les produits cosmétiques, et ainsi éviter les problèmes d'incompatibilité.
La présente invention a donc pour objet une composition cosmétique comprenant, dans un milieu cosmétiquement acceptable, un composé susceptible d'être obtenu par réaction entre :
- au moins une cire portant au moins une fonction réactive choisie parmi OH ou COOH, la cire étant choisie parmi la cire de Candellila, la cire de Carnauba, la cire de canne à sucre, la cire d'abeille, la cire de Montan, la cire de jojoba; et
- au moins un groupe de jonction capable d'établir des liaisons hydrogène avec un ou plusieurs groupes de jonction partenaires, chaque appariement d'un groupe de jonction faisant intervenir au moins 4 liaisons hydrogène, ledit groupe de jonction portant au moins une fonction réactive dite complémentaire, susceptible de réagir avec la fonction réactive portée par la cire, ledit groupe de jonction comprenant au moins un motif de formule (I) ou (II) telle que définie ci-après.
Le composé ainsi obtenu forme un autre objet de l'invention.

En fonctionnalisant la cire par des uréidopyrimidones, il est possible d'obtenir un dépôt homogène sur les matières kératiniques, voire avec des propriétés proches d'un film, et ainsi permettre soit l'utilisation de faible quantité de cires pour un durcissement équivalent, soit une utilisation à haute concentration sans avoir un effet collant ou gras à l'application.
Les cires fonctionnalisées selon la présente invention se présentent sous forme d'un solide; ceci permet notamment de former un matériau non collant, qui ne transfère pas au doigt une fois appliqué sur un substrat kératinique.
Par ailleurs, on a constaté que la réticulation à travers quatre liaisons hydrogène, par l'intermédiaire des groupes uréïdopyrimidone, pouvait permettre d'augmenter la force de cette réticulation, et donc améliorer la tenue de l'effet cosmétique recherché, tout particulièrement la tenue du dépôt.
De plus, les composés, ou cires fonctionnalisées, selon l'invention sont aisément véhiculables dans les milieux cosmétiques usuels, notamment les milieux huileux cosmétiques usuels.
Ils sont aisément véhiculables dans les milieux solvants ou huileux cosmétiques, notamment les huiles, les alcools gras et/ou les esters gras, ce qui facilite leur mise en oeuvre dans le domaine cosmétique, notamment dans le maquillage. Ils présentent une solubilité convenable dans des milieux huileux cosmétiques variés, tels que les huiles végétales, les alcanes, les esters qu'ils soient courts de type acétate de butyle ou d'éthyle, ou gras, les alcools gras et tout particulièrement dans les milieux comprenant de l'isododécane, du Parléam, de l'isononanoate d'isononyle, de l'octyldodécanol, et/ou un benzoate d'alkyle en C12-C15.

Les composés selon l'invention sont susceptibles d'être obtenus par réaction entre :
- d'une part au moins une cire portant au moins une fonction réactive choisie parmi OH ou COOH, la cire étant choisie parmi la cire de Candellila, la cire de Carnauba, la cire de canne à sucre, la cire d'abeille, la cire de Montan, la cire de jojoba; et
- d'autre part au moins un groupe de jonction capable d'établir des liaisons hydrogène avec un ou plusieurs groupes de jonction partenaires, chaque appariement d'un groupe de jonction faisant intervenir au moins 4 liaisons hydrogène, ledit groupe de jonction portant au moins une fonction réactive dite complémentaire, susceptible de réagir avec la fonction réactive portée par la cire, notamment isocyanate, ledit groupe de jonction comprenant au moins un motif de formule (I) ou (II) telle que définie ci-après.
Au final, les composés selon l'invention comportent donc au moins une partie provenant de la cire et au moins une partie (G) provenant du groupe de jonction, ladite partie (G) comprenant au moins un motif de formule (I) ou (II).
Notamment, lesdites parties sont reliées par une liaison covalente, notamment peuvent être reliées par une liaison covalente formée lors de la réaction entre les fonctions réactives OH et/ou COOH portées par la cire et les fonctions réactives complémentaires, notamment isocyanate, portées par le groupe de jonction, susceptibles de réagir avec lesdites fonctions OH et COOH.
La cire susceptible d'être utilisée dans le cadre de la présente invention porte donc au moins une fonction réactive susceptible de réagir avec la fonction réactive complémentaire portée par le groupe de jonction, notamment susceptible de réagir chimiquement avec les groupes isocyanate portés par le groupe de jonction. Selon l'invention, cette fonction est une fonction OH ou COOH. De préférence, la cire ne porte que des fonctions OH, préférentiellement des fonctions OH primaire ou secondaires, et encore mieux uniquement primaires.
La cire susceptible d'être employée pour préparer le composé selon l'invention, est un corps gras lipophile ou un mélange de corps gras lipophile, cristallin à 25°C, solide à température ambiante et sous pression atmosphérique (25°C, 1 atm.); de préférence à changement d'état solide/liquide réversible, et ayant généralement une température de fusion supérieure à 40°C, mieux supérieure à 55°C, et encore mieux supérieure à 75°C, et pouvant aller jusqu'à 200°C, notamment jusqu'à 120°C. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange. Les cires susceptibles d'être employées dans la présente invention sont choisies parmi la cire de Candellila, la cire de canne à sucre, la cire de Montan, la cire d'abeille, la cire de Carnauba et la cire de jojoba, et leurs mélanges.

Le groupe de jonction susceptible d'être utilisé pour former le composé selon l'invention porte au moins une fonction réactive complémentaire aux fonctions OH ou COOH portées par la cire, notamment une fonction isocyanate. Cette fonction est susceptible de réagir avec les fonctions réactives OH ou COOH de la cire afin de former une liaison covalente, notamment de type uréthanne, entre ladite cire et ledit groupe de jonction.
Ledit groupe de jonction est capable d'établir des liaisons H avec un ou plusieurs groupes de jonction partenaires, de nature chimique identique ou différente, chaque appariement d'un groupe de jonction faisant intervenir au moins 4 liaisons H (hydrogène).

Par "groupe de jonction", on entend au sens de l'invention, tout groupe fonctionnel comportant des groupes donneurs ou accepteurs de liaisons H, et capable d'établir au moins trois liaisons H, de préférence au moins 4 liaisons H, préférentiellement 4 liaisons H, avec un groupe de jonction partenaire, identique ou non. Par " groupe de jonction partenaire", on entend au sens de l'invention, tout groupe de jonction pouvant établir des liaisons H avec un ou plusieurs groupes de jonction d'un même ou d'un autre polymère selon l'invention. Les groupes de jonction peuvent être de nature chimique identique ou différente. S'ils sont identiques, ils peuvent alors établir des liaisons H entre eux et sont alors appelés groupes de jonction auto-complémentaires. S'ils sont différents, ils sont choisis de telle façon qu'ils soient complémentaires vis à vis des interactions H.

Ledit groupe de jonction, porteur de groupes isocyanates, peut donc être schématisé (G)-(NCO)ₚ, p étant un entier non nul, de préférence égal à 1 ou 2.

Le groupe de jonction comprend par ailleurs au moins un motif monovalent de formule (I) et/ou au moins un motif divalent de formule (II), telles que ci-dessous définies: 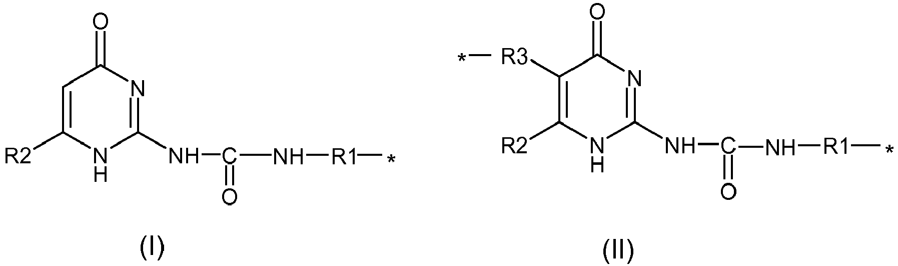 dans lesquelles :
- R1 et R3, identiques ou différents, représentent un radical carboné divalent choisi parmi (i) un groupe alkyle linéaire ou ramifié en C₁-C₃₂, (ii) un groupe cycloalkyle en C₄-C₁₆ et (iii) un groupe aryle en C₄-C₁₆; comprenant éventuellement 1 à 8 hétéroatomes choisis parmi O, N, S, F, Si et P; et/ou éventuellement substitué par une fonction ester, amide ou par un radical alkyle en C₁-C₁₂; ou un mélange de ces groupes;
- R2 représente un atome d'hydrogène ou un radical carboné, notamment hydrocarboné (alkyle), linéaire, ramifié ou cyclique, saturé ou insaturé, éventuellement aromatique, en C1-C32, pouvant comprendre un ou plusieurs hétéroatomes choisis parmi O, N, S, F, Si et P.

Notamment, le radical R1 peut notamment être :
- un groupe alkylène divalent, linéaire ou ramifié, en C2-C12, notamment un groupe 1,2-éthylène, 1,6-hexylène, 1,4-butylène, 1,6-(2,4,4-triméthylhexylène), 1,4-(4-méthylpentylène), 1,5-(5-méthylhexylène), 1,6-(6-méthylheptylène), 1,5-(2,2,5-triméthylhexylène), 1,7-(3,7-diméthyloctylène).
- un groupe cycloalkylène ou arylène, divalent, en C4-C12, notamment choisi parmi les radicaux suivants -isophorone-, tolylène, 2-méthyl-1,3-phénylène, 4-méthyl-1,3-phénylène; 4,4'-méthylènebiscyclohexylène; 4,4-bisphénylèneméthylène; ou de structure : 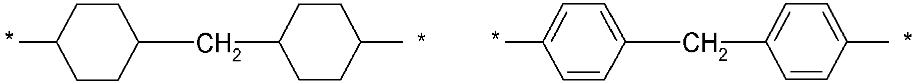
Par -isohorone- on entend le radical divalent de structure : 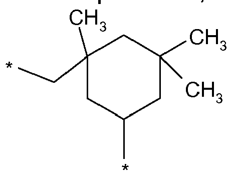 Préférentiellement, R1 représente -isophorone-, -(CH₂)₆- ou 4,4'-méthylènebiscyclohexylène.

Notamment, le radical R2 peut notamment être H, ou bien :
- un groupe alkyle en C₁-C₃₂, en particulier en C1-C16, voire en C1-C10;
- un groupe cycloalkyle en C₄-C₁₂ ;
- un groupe aryle en C₄-C₁₂ ;
- un groupe aryl(C₄-C₁₂) alkyle en C₁-C₁₈
- un groupe alcoxy en C₁-C₄ ;
- un groupe arylalcoxy, en particulier un groupe aryle (C₁-C₄) alcoxy ;
- un hétérocycle en C₄-C₁₂
ou une combinaison de ces radicaux, qui peuvent éventuellement être substitués par une fonction amino, ester et/ou hydroxy.
De préférence R2 représente H, CH₃, éthyle, C₁₃H₂₇, C₇H₁₅, phényle, isopropyle, isobutyle, n-butyle, tert-butyle, n-propyle, ou encore -CH(C₂H₅)(C₄H₉).

De préférence, R3 représente un radical divalent -R'3-O-C(O)-NH-R'4- dans lequel R'3 et R'4, identiques ou différents, représentent un radical carboné divalent choisi parmi un groupe alkyle linéaire ou ramifié en C₁-C₃₂ ou un groupe cycloalkyle en C₄-C₁₆ ou un groupe aryle en C₄-C₁₆; ou leur mélange.
En particulier, R'3 et R'4 peuvent représenter méthylène, 1,2-éthylène, 1,6-hexylène, 1,4-butylène, 1,6-(2,4,4-triméthylhexylène), 1,4-(4-méthylpentylène), 1,5-(5-méthylhexylène); 1,6-(6-méthylheptylène); 1,5-(2,2,5-triméthylhexylène), 1,7-(3,7-diméthyloctylène); 4,4'-méthylènebiscyclohexylène; 2-méthyl-1,3-phénylène; 4-méthyl-1,3-phénylène; 4,4'-bisphénylèneméthylène; 1,2-tolylène, 1,4-tolylène, 2,4-tolylène, 2,6-tolylène; 1,5-naphtylène; tétraméthylxylylène; isophorone.
Tout particulièrement, R'3 peut représenter un alkylène en C1-C4, notamment 1,2-éthylène.
De préférence, R'4 peut représenter le radical divalent dérivé de l'isophorone.

Tout particulièrement, R3 peut être de structure :

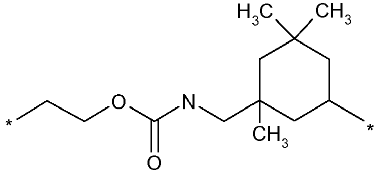

De façon particulièrement préférée, dans la formule (I), on peut avoir :
- R₁ = -isophorone-, R2 = méthyl, ce qui conduit au motif de formule : 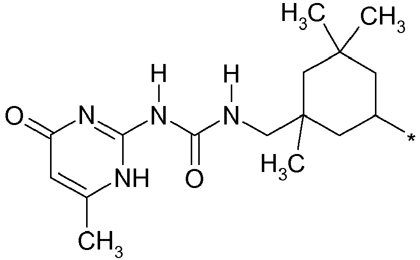
- R₁ = -(CH₂)₆-, R2 = méthyl, ce qui conduit au motif de formule : 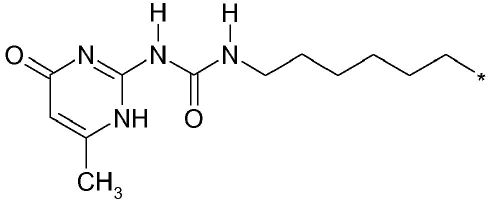
- R₁ = -(CH₂)₆-, R2 = isopropyl, ce qui conduit au motif de formule : 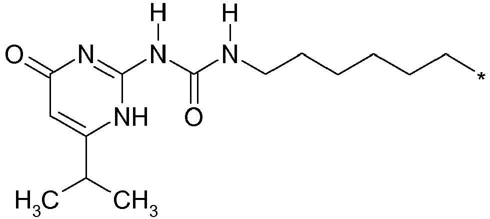
- R₁ = 4,4'-méthylènebiscyclohexylène et R2 = méthyle, ce qui conduit au motif de formule :

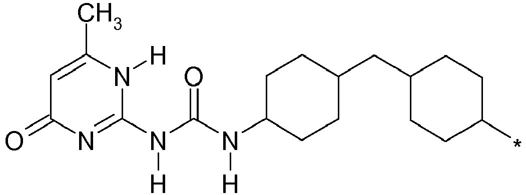

De façon particulièrement préférée, dans la formule (II), R1 représente le radical - isophorone-, R2= méthyle et R3=-(CH₂)₂OCO-NH-isophorone-, ce qui conduit au motif divalent de formule : 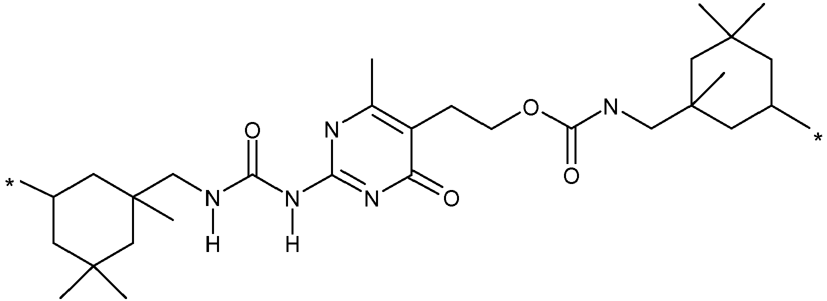 Les groupes de jonction porteurs d'une seule fonction isocyanate peuvent être de formule : 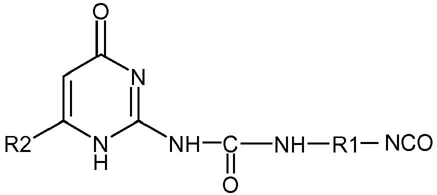 dans laquelle R1 et R2 sont tels que définis ci-dessus; et en particulier :
- R1 représente -isophorone-, -(CH₂)₆-, -CH₂CH(CH₃)-CH₂-C(CH₃)₂-CH₂-CH₂, 4,4'-méthylènebiscyclohexylène, 2-méthyl-1,3-phénylène; et/ou
- R2 représente H, CH₃, éthyle, C₁₃H₂₇, C₇H₁₅, phényle, isopropyle, isobutyle, n-butyle, tert-butyle, n-propyle, ou encore -CH(C₂H₅)(C₄H₉).

De manière préférée, les groupes de jonction peuvent être choisis parmi les groupes suivants :

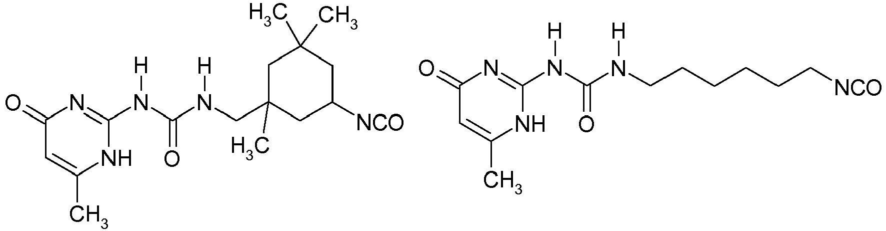

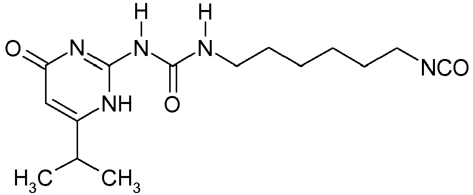

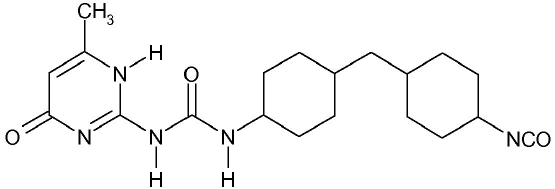

Les groupes de jonction porteurs de deux fonctions isocyanate peuvent être de formule : 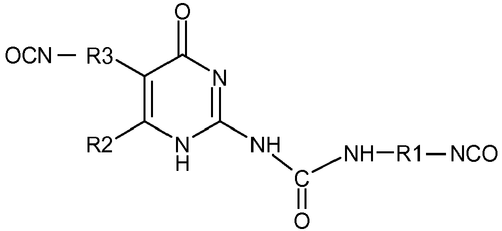 dans laquelle R1, R2 et R3 sont tels que définis ci-dessus, et en particulier :
- R1 représente -isophorone-, -(CH₂)₂-, -(CH₂)₆-, -CH₂CH(CH₃)-CH₂-C(CH₃)₂-CH₂-CH₂, 4,4'-méthylènebiscyclohexylène, 2-méthyl-1,3-phénylène; et/ou
- R2 représente H, CH₃, éthyle, C₁₃H₂₇, C₇H₁₅, phényle, isopropyle, isobutyle, n-butyle, tert-butyle, n-propyle, ou encore -CH(C₂H₅)(C₄H₉); et/ou
- R3 représente un radical divalent -R'3-O-C(O)-NH-R'4- dans lequel R'3 et R'4, identiques ou différents, représentent un radical carboné divalent choisi parmi un groupe alkyle linéaire ou ramifié en C₁-C₃₀ ou un groupe cycloalkyle en C₄-C₁₂ ou un groupe aryle en C₄-C₁₂; ou leur mélanges; et notamment R'3 représente un alkylène en C1-C4, notamment 1,2-éthylène et R'4 représente le radical divalent dérivé de l'isophorone.

Un groupe de jonction tout particulièrement préféré est celui de formule : 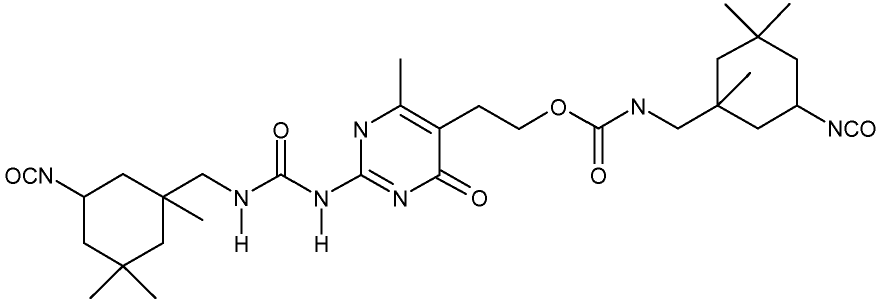 Selon un mode particulier de réalisation de l'invention, les groupes de jonction peuvent être fixés sur la cire via la fonctionnalisation du groupe de jonction par un isocyanate. Selon un autre mode de réalisation, il est possible de faire la réaction inverse en préfonctionnalisant la cire par un diisocyanate.

Ainsi que mentionné ci-dessus, le composé selon l'invention peut donc résulter de la réaction chimique entre une cire et un groupe de jonction.
Le composé selon l'invention peut être préparé par les procédés usuellement employés par l'homme du métier pour former une liaison uréthanne, entre les fonctions OH libres de la cire et les fonctions isocyanates portées par le groupe de jonction. A titre d'illustration, un procédé général de préparation consiste à :
- chauffer la cire comportant au moins une fonction réactive, notamment OH, à une température pouvant être comprise entre 60°C et 140°C;
- ajouter le groupe de jonction portant les fonctions réactives, notamment isocyanate;
- éventuellement agiter le mélange, sous atmosphère contrôlée, à une température de l'ordre de 100-130°C; pendant 1 à 24 heures;
- suivre par spectroscopie infrarouge, la disparition de la bande caractéristique des isocyanates (compris entre 2500 et 2800 cm-1) de manière à arrêter la réaction à la disparition totale du pic, puis à laisser revenir à température ambiante le produit final.
La réaction peut être effectuée en présence d'un solvant, notamment la méthyltétrahydrofurane, le tétrahydrofurane, le toluène ou l'acétate de butyle. Il est également possible d'ajouter un catalyseur conventionnel de la formation de liaison uréthane. A titre d'exemple, on peut citer le dilaurate dibutyle étain. Le composé peut au final être lavé et séché, voire purifié, selon les connaissances générales de l'homme du métier.

Selon un autre mode de réalisation, la réaction peut comporter les étapes suivantes fonctionnalisation de la cire par un diisocyanate, puis réaction avec la 6-méthylisocytosine ou la 5-hydroxyéthyl-6-méthyl isocytosine. Une illustration d'une telle réaction est donnée dans FOLMER et al., Adv. Mater, 12, 874-78 (2000).

On a constaté que l'utilisation des composés selon l'invention peut conduire, après application de la composition sur les matières kératiniques, à la formation d'un polymère supramoléculaire sous forme de réseau réticulé physiquement, notamment à travers des liaisons hydrogène, se présentant généralement sous forme de film, et ayant une très bonne résistance mécanique.
Par "polymère supramoléculaire", on entend au sens de l'invention, une chaîne ou un réseau polymérique formé de l'assemblage de composés non polymères selon l'invention avec au moins un autre composé non polymère selon l'invention, identique ou différent, chaque assemblage comprenant au moins une paire de groupes de jonction appariés, identiques ou différents.
Par "paire de groupes de jonction appariés", on entend au sens de l'invention, deux groupes de jonction dont chacun peut être porté ou non par un même composé selon l'invention, les deux groupes étant reliés ensemble via 4 liaisons H.
Ainsi le polymère supramoléculaire présentera des points de réticulation physique assurés par les liaisons H entre ces paires de groupes de jonction. La réticulation physique assurera le maintien et la persistance de l'effet cosmétique de manière analogue à la réticulation chimique, tout en permettant la réversibilité, c'est-à-dire la possibilité d'éliminer totalement le dépôt.

La masse moléculaire moyenne en nombre (Mn) du composé selon l'invention est de préférence comprise entre 180 à 18 000, de préférence 200 à 15 000, voire de 300 à 10 000, et encore mieux de 400 à 5000, préférentiellement de 500 à 2500.

Le composé selon l'invention est avantageusement soluble dans les milieux huileux cosmétiques usuellement employés, et notamment dans les huiles végétales, les alcanes en C6-C32, les esters gras en C8-C32, les esters courts en C2-C7, les alcools gras en C8-C32, et plus particulièrement dans les milieux comprenant au moins de l'isododécane, du Parléam, de l'isononanoate d'isononyle, de l'octyldodécanol, du benzoate d'alkyle en C12-C15, de l'acétate de butyle, de l'acétate d'éthyle, seul ou en mélange. Par soluble, on entend que le composé forme une solution limpide dans au moins un solvant choisi parmi l'isododécane, le Parléam, l'isononanoate d'isononyle, l'octyldodécanol, le benzoate d'alkyle en C12-C15, l'acétate de butyle, l'acétate d'éthyle, à raison d'au moins 5% en poids, à 25°C.

Les composés selon l'invention peuvent être utilisés avantageusement dans une composition cosmétique qui comprend par ailleurs un milieu cosmétiquement acceptable, c'est-à-dire compatible avec les matières kératiniques telles que la peau du visage ou du corps, les cils, les sourcils, les lèvres et les ongles.
La quantité de composé présent dans les compositions dépend bien entendu du type de composition et des propriétés recherchées et peut varier à l'intérieur d'une gamme très large, comprise généralement entre 5 et 80% en poids, de préférence entre 10 et 75% en poids, notamment entre 20 et 70% en poids, voire entre 25 et 65% en poids, et mieux entre 30 et 60% en poids, par rapport au poids de la composition cosmétique finale.

La composition peut alors comprendre, selon l'application envisagée, les constituants habituels à ce type de composition.
La composition selon l'invention peut avantageusement comprendre une phase grasse liquide, qui peut constituer un milieu solvant des polymères selon l'invention, et qui peut comprendre au moins un composé choisi parmi les huiles et/ou solvants d'origine minérale, animale, végétale ou synthétique, carbonés, hydrocarbonés, fluorés et/ou siliconés, volatils ou non volatils, seuls ou en mélange dans la mesure où ils forment un mélange homogène et stable et sont compatibles avec l'utilisation envisagée.
Par 'volatil', on entend au sens de l'invention, tout composé susceptible de s'évaporer au contact des matières kératiniques, ou des lèvres, en moins d'une heure, à température ambiante (25°C) et pression atmosphérique (1 atm). Notamment, ce composé volatil a une pression de vapeur non nulle, à température ambiante et pression atmosphérique, notamment allant de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).
Par opposition, on entend par 'non volatil', un composé restant sur les matières kératiniques ou les lèvres à température ambiante et pression atmosphérique, au moins une heure et ayant notamment une pression de vapeur inférieure à 10⁻³ mm de Hg (0,13Pa).

De préférence, le milieu physiologiquement acceptable de la composition selon l'invention peut comprendre, dans une phase grasse liquide, au moins une huile et/ou un solvant qui peut être choisi parmi, seul ou en mélange :
1/ les esters des acides monocarboxyliques avec les monoalcools et polyalcools; avantageusement, ledit ester est un benzoate d'alkyle en C12-C15 ou répond à la formule suivante : R'₁-COO-R'₂ où :
   R'₁ représente un radical alkyle linéaire ou ramifié de 1 à 40 atomes de carbone, de préférence de 7 à 19 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles, et
   R'₂ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles.

   Par "éventuellement substitué", on entend que R'₁ et/ou R'₂ peuvent porter un ou plusieurs substituants choisis, par exemple, parmi les groupements comprenant un ou plusieurs hétéroatomes choisi parmi O et/ou N, tels que amino, amine, alcoxy, hydroxyle.
   Des exemples des groupes R'₁ sont ceux dérivés des acides gras de préférence supérieur choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, oléostéarique, arachidonique, érucique, et de leurs mélanges. De préférence, R'₁ est un groupe alkyle ramifié non substitué de 4 à 14 atomes de carbone, de préférence de 8 à 10 atomes de carbone et R₂ est un groupe alkyle ramifié non substitué de 5 à 15 atomes de carbone, de préférence de 9 à 11 atomes de carbone.
   On peut en particulier citer, de préférence, les esters en C₈-C₄₈, éventuellement incorporant dans leur chaîne hydrocarbonée un ou plusieurs hétéroatomes parmi N et O et/ou une ou plusieurs fonctions carbonyle; et plus particulièrement l'huile de purcellin (octanoate de cétostéaryle), l'isononanoate d'isononyle, le myristate d'isopropyle, le palmitate d'isopropyle, le palmitate d'éthyl-2-hexyle, le stéarate d'octyl-2-dodécyle, l'érucate d'octyl-2-dodécyle, l'isostéarate d'isostéaryle, le benzoate d'alcool en C₁₂ à C₁₅, le laurate d'hexyle, l'adipate de diisopropyle; et les heptanoates, octanoates, décanoates ou ricinoléates d'alcools ou de polyalcools, par exemple d'alcools gras comme le dioctanoate de propylène glycol, ainsi que le N-lauroyl sarcosinate d'isopropyle (notamment Eldew-205SL d'Ajinomoto); les esters hydroxylés comme le lactate d'isostéaryle, le malate de di-isostéaryle; et les esters du pentaérythritol; les esters ramifiés en C8-C16, notamment le néopentanoate d'isohexyle.
2/ les huiles végétales hydrocarbonées à forte teneur en triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat, de jojoba, de palme, de calophyllum; ou encore les triglycérides des acides caprylique/caprique comme ceux vendus par la société Stearinerie Dubois ou ceux vendus sous les dénominations "Miglyol 810®", "812® " et "818® " par la société Dynamit Nobel.
3/ les alcools, et notamment les monoalcools, en C6-C32, notamment C12-C26, comme l'alcool oléïque, l'alcool linoléique, l'alcool linolénique, l'alcool isostéarylique, le 2-hexyldécanol, le 2-butyloctanol, le 2-undécylpentadécanol et l'octyldodécanol;
4/ les huiles hydrocarbonées, linéaires ou ramifiés, volatiles ou non, d'origine synthétique ou minérale, qui peuvent être choisies parmi les huiles hydrocarbonées ayant de 5 à 100 atomes de carbones, et notamment la vaseline, les polydécènes, les polyisobutènes hydrogénés tel que le Parléam, le squalane, le perhydrosqualène et leurs mélanges.
   On peut plus particulièrement citer les alcanes linéaires, ramifiés et/ou cycliques en C5-C48, et préférentiellement les alcanes ramifiés en C8-C16 comme les isoalcanes en C8-C16 d'origine pétrolière (appelées aussi isoparaffines); notamment le décane, l'heptane, le dodécane, le cyclohexane; ainsi que l'isododécane, l'isodécane, l'isohexadécane.
5/ les huiles de silicone, volatiles ou non volatiles;
   Comme huiles de silicone volatiles, on peut citer les huiles de silicones linéaires ou cycliques volatiles, notamment celles ayant une viscosité inférieure à 8 centistokes, et ayant notamment de 2 à 10 atomes de silicium, ces silicones comportant éventuellement des groupes alkyles ou alkoxy ayant de 1 à 22 atomes de carbone; et en particulier l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'hexaméthyldisiloxane, l'octaméthyltrisiloxane, le décaméthyltétrasiloxane, le dodécaméthylpentasiloxane, le méthylhexyldiméthylsiloxane et leurs mélanges.
Les huiles de silicone non volatiles utilisables selon l'invention peuvent être les polydiméthylsiloxanes (PDMS), les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényltriméthicones, les phényldiméthicones, les phényltriméthylsiloxydiphénylsiloxanes, les diphényldiméthicones, les diphénylméthyldiphényltrisiloxanes, les 2-phényléthyl triméthylsiloxysilicates.

Préférentiellement, le milieu physiologiquement acceptable de la composition selon l'invention comprend, dans une phase grasse liquide, au moins une huile et/ou un solvant choisi parmi, seul ou en mélange, l'isododécane, le Parléam, l'isononanoate d'isononyle, l'octyldodécanol, la phényl triméthicone, les benzoates d'alkyle en C12-C15, les acétates de butyle et d'éthyle, et/ou la D5 (décaméthylcyclopentasiloxane).

La phase grasse liquide peut en outre comprendre des huiles et/ou solvants additionnels, qui peuvent être choisis parmi, seul ou en mélange :
- les huiles fluorées telles que les perfluoropolyéthers, les perfluoroalcanes comme la perfluorodécaline, les perfluorodamantanes, les monoesters, diesters et triesters de perfluoroalkylphosphates et les huiles esters fluorés;
- les huiles d'origine animale;
- les éthers en C₆ à C₄₀, notamment en C10-C40; les éthers de propylène glycol liquides à température ambiante tels que le monométhyléther de propylène glycol, l'acétate de monométhyléther de propylène glycol, le mono n-butyl éther de dipropylène glycol ;
- les acides gras en C₈-C₃₂, comme l'acide oléique, l'acide linoléique, l'acide linolénique et leurs mélanges.
- les huiles bifonctionnelles, comprenant deux fonctions choisies parmi ester et/ou amide et comprenant de 6 à 30 atomes de carbone, notamment 8 à 28 atomes de carbone, mieux de 10 à 24 carbones, et 4 hétéroatomes choisis parmi O et N; de préférence les fonctions amide et ester étant dans la chaîne;
- les cétones liquides à température ambiante (25°C) tels que la méthyléthylcétone, la méthylisobutylcétone, la diisobutylcétone, l'isophorone, la cyclohexanone, l'acétone ;
- les aldéhydes liquides à température ambiante tels que le benzaldéhyde, l'acétaldéhyde;
La phase grasse liquide peut représenter 1 à 90% en poids de la composition, notamment de 5 à 75% en poids, en particulier de 10 à 60% en poids, voire de 25 à 55% en poids, du poids total de la composition.

La composition selon l'invention peut comprendre avantageusement un agent épaississant qui peut en particulier être choisi parmi :
- les silices notamment hydrophobes, telles que celles décrites dans le document EP-A-898960, et par exemple commercialisées sous les références "AEROSIL R812®" par la société Degussa, "CAB-O-SIL TS-530®", "CAB-O-SIL TS-610®", "CAB-O-SIL TS-720®"par la société Cabot, "AEROSIL R972®", "AEROSIL R974®" par la société Degussa ;
- les argiles telles que la montmorillonite, les argiles modifiées telles que les bentones par exemple, l'hectorite stéaralkonium, la bentonite stéaralkonium,
- les alkyléther de polysaccharides (notamment dont le groupe alkyle comporte de 1 à 24 atomes de carbones, de préférence de 1 à 10, mieux de 1 à 6, et plus spécialement de 1 à 3) tels que ceux décrits dans le document EP-A-898958.
La quantité d'agent épaississant dans la composition selon l'invention peut aller de 0,05 à 40% en poids, par rapport au poids total de la composition, de préférence de 0,5 à 20% et mieux de 1 à 15% en poids.

La composition selon l'invention peut également comprendre au moins une cire d'origine végétale, animale, minérale ou de synthèse, voire siliconée.
On peut en particulier citer, seules ou en mélange, les cires hydrocarbonées telles que la cire d'abeilles; la cire de Carnauba, de Candellila, d'Ouricoury, du Japon, les cires de fibres de lièges ou de canne à sucre; les cires de paraffine, de lignite; les cires microcristallines; la cire de lanoline; la cire de Montan; les ozokérites; les cires de polyéthylène; les cires obtenues par synthèse de Fischer-Tropsch; les huiles hydrogénées, les esters gras et les glycérides concrets à 25°C. On peut également utiliser des cires de silicone, parmi lesquelles on peut citer les alkyls, alcoxys et/ou esters de polyméthylsiloxane.
La quantité de cire dans la composition selon l'invention peut aller de 0,1 à 70% en poids, par rapport au poids total de la composition, de préférence de 1 à 40% en poids, et mieux de 5 à 30% en poids.

La composition selon l'invention peut également comprendre une ou plusieurs matières colorantes choisies parmi les composés pulvérulents comme les pigments, les charges, les nacres et les paillettes, et/ou les colorants liposolubles ou hydrosolubles.
Les matières colorantes, notamment pulvérulentes, peuvent être présentes, dans la composition, en une teneur de 0,01 à 50% en poids, par rapport au poids de la composition, de préférence de 0,1 à 40% en poids, voire de 1 à 30% en poids.
Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.
Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.
Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.
Les charges peuvent être minérales ou organiques, lamellaires ou sphériques. On peut citer le talc, le mica, la silice, le kaolin, les poudres de Nylon et de polyéthylène, de poly-β-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.
Les colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. Ils peuvent représenter 0,01 à 20% du poids de la composition et mieux de 0,1 à 6 %.
Les colorants hydrosolubles sont par exemple le jus de betterave, le bleu de méthylène et peuvent représenter 0,01 à 6% du poids total de la composition.

La composition peut comprendre, en outre, d'autres ingrédients utilisés couramment dans les compositions cosmétiques. De tels ingrédients peuvent être choisis parmi les antioxydants, les parfums, les huiles essentielles, les conservateurs, les actifs cosmétiques, les hydratants, les vitamines, les céramides, les filtres solaires, les tensioactifs, les gélifiants, les agents d'étalement, les agents mouillants, les agents dispersants, les anti-mousses, les neutralisants, les stabilisants, les polymères et notamment les polymères filmogènes liposolubles, et leurs mélanges.
Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Les compositions selon l'invention peuvent se présenter sous toute forme acceptable et usuelle pour une composition cosmétique. Elles peuvent donc se présenter sous la forme d'une suspension, une dispersion notamment d'huile dans de l'eau grâce à des vésicules; une solution organique ou huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphasé ou multiphase; un spray; d'une lotion, d'une crème, d'une pommade, d'une pâte souple, d'un onguent, d'un solide coulé ou moulé et notamment en stick ou en coupelle, ou encore de solide compacté.
L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Les compositions conformes à l'invention peuvent être utilisées pour le soin ou le maquillage des matières kératiniques telles que la peau, les cils, les sourcils, les ongles, les lèvres, les cheveux, et plus particulièrement pour le maquillage des lèvres, des cils et/ou du visage.
Elles peuvent donc se présenter sous la forme d'un produit de soin et/ou de maquillage de la peau du corps ou du visage, des lèvres, des cils, des sourcils, des cheveux ou des ongles; d'un produit solaire ou autobronzant; d'un produit capillaire; elles se présentent avantageusement sous forme de composition de maquillage, notamment de mascara, d'eye-liner, de rouge à lèvres, de brillant à lèvres (gloss), de fard à joues ou à paupières, de fond de teint, de vernis à ongles ou de soin des ongles.

L'invention a encore pour objet un procédé de traitement cosmétique des matières kératiniques, notamment de la peau du corps ou du visage, des lèvres, des ongles, des cils et/ou des cheveux, comprenant l'application sur lesdites matières d'une composition cosmétique telle que définie précédemment.
Ce procédé selon l'invention permet notamment le soin ou le maquillage desdites matières kératiniques, en particulier des lèvres et/ou des ongles, par application d'une composition notamment de rouge à lèvres, de fond de teint, de mascara selon l'invention.

L'invention est illustrée plus en détail dans les exemples de réalisation suivants.

### Exemple 1

On chauffe 13 g de cire d'abeille en mélange avec 11 mg de dibutylétain dilaurate (catalyseur) et 0,93 g de méthylisocytosine. Le mélange est chauffé à 100°C et mis sous vide pendant 2 heures. Le mélange est ensuite descendu à 70°C, sous argon et on ajoute 1,5g d'isophorone diisocyanate; on mélange en laissant sous argon pendant 2 heures à 70°C.
On ajoute ensuite 2,5 ml de propylène carbonate anhydre, et la température du milieu réactionnel est montée à 140°C, sous argon et sous agitation pendant 3 heures à cette température. La disparition des fonctions isocyanates est suivie par spectroscopie infrarouge, jusqu'à disparition complète du pic caractéristique à 2250 cm⁻¹. La température du mélange est ensuite ramenée à 70°C, et on ajoute 60 ml de tétrahydrofurane et 5 ml d'éthanol, suivi d'une agitation pendant 30 minutes. Le mélange réactionnel est filtré sur célite, précipité dans l'éthanol et séché sous pression réduit.
On obtient la cire fonctionnalisée recherchée, sous forme d'une cire blanche.

### Observations :

- cire d'abeille à 23% dans l'isododécane : le mélange est blanc et compact; après dépôt à chaud sur une plaque de verre et refroidissement à 25°C, on observe qu'il forme un dépôt blanc collant, non cohésif. Au toucher, le doigt s'enfonce dans le dépôt; le toucher est gras.
- cire d'abeille fonctionnalisée préparée ci-dessus, à 23% dans l'isododécane : le mélange est blanc et compact; après dépôt à chaud sur une plaque de verre et refroidissement à 25°C, on observe qu'il forme un dépôt non collant et cassant après évaporation de l'isododécane. On n'observe aucune sensation de gras au toucher; au contraire, le toucher est sec; le doigt ne s'enfonce pas dans le dépôt.

### Exemple 2

On prépare un rouge à lèvres comprenant (% en poids):
- cire d'abeille fonctionnalisée préparée à l'exemple 1 10%
- isododécane 40%
- cyclopentapolysiloxane 10%
- polyisobutène hydrogéné 30%
- pigments 10%

### Exemple 3 (hors invention)

On chauffe 19,3 g d'alcool cétylique sous pression réduite, à 60°C, et après deux heures, on ajoute 10 ml d'acétate de butyle anhydre; le mélange est amené à 40°C, toujours sous atmosphère contrôlée. On ajoute ensuite 18,9 g d'IPDI (isophorone diisocyanate) puis 10 ml supplémentaire d'acétate de butyle, et 9 mg de catalyseur (dilaurate dibutyl étain). Le mélange réactionnel est agité pendant 16 heures à 40°C, sous atmosphère contrôlée. On ajoute alors 12,73 g de méthyl isocytosine et 20 ml de propylène carbonate anhydre, et le mélange est porté à 140°C, température maintenue pendant 2h30. La disparition des isocyanates est suivie par spectroscopie IR, et, après la totale disparition du pic caractéristique des isocyanates (2250 cm⁻¹), l'agitation est maintenue 30 minutes supplémentaires. Le milieu réactionnel est amené à 70°C, dilué avec 200 ml d'hexane et filtré sur célite. Cette phase organique est lavée trois fois avec un mélange H₂O/éthanol (2/1) saturé en NaCl, puis séchée sur Na₂SO4, filtrée et séchée sous pression réduite. On obtient le produit recherché sous forme d'un solide cassant.

## Revendications

1. Composition cosmétique comprenant, dans un milieu cosmétiquement acceptable, un composé susceptible d'être obtenu par réaction entre :
- au moins une cire portant au moins une fonction réactive choisie parmi OH ou COOH, la cire étant choisie parmi la cire de Candellila, la cire de Carnauba, la cire de canne à sucre, la cire d'abeille, la cire de Montan, la cire de jojoba ;
et
- au moins un groupe de jonction capable d'établir des liaisons hydrogène avec un ou plusieurs groupes de jonction partenaires, chaque appariement d'un groupe de jonction faisant intervenir au moins 4 liaisons hydrogène, ledit groupe de jonction portant au moins une fonction réactive dite complémentaire, susceptible de réagir avec la fonction réactive portée par la cire, ledit groupe de jonction comprenant au moins un motif de formule (I) ou (II) : 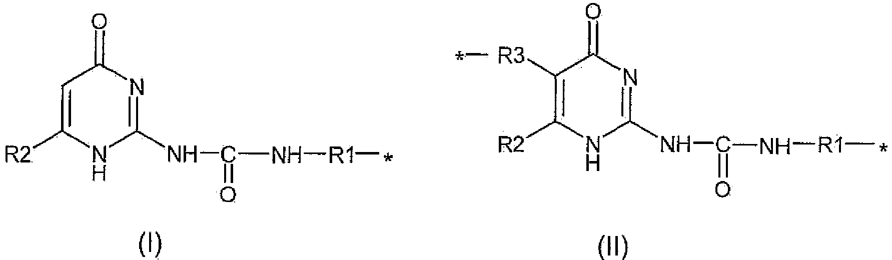 dans lesquelles :
- R1 et R3, identiques ou différents, représentent un radical carboné divalent choisi parmi (i) un groupe alkyle linéaire ou ramifié en C₁-C₃₂, (ii) un groupe cycloalkyle en C₄-C₁₆ et (iii) un groupe aryle en C₄-C₁₆; comprenant éventuellement 1 à 8 hétéroatomes choisis parmi O, N, S, F, Si et P; et/ou éventuellement substitué par une fonction ester, amide ou par un radical alkyle en C₁-C₁₂; ou un mélange de ces groupes;
- R2 représente un atome d'hydrogène ou un radical carboné, notamment hydrocarboné (alkyle), linéaire, ramifié ou cyclique, saturé ou insaturé, éventuellement aromatique, en C₁-C₃₂, pouvant comprendre un ou plusieurs hétéroatomes choisis parmi O, N, S, F, Si et P.

2. Composition selon la revendication 1, dans laquelle la cire portant au moins une fonction réactive, notamment OH, COOH, est choisie parmi, seule ou en mélange :
la cire d'abeille, la cire de Carnauba et la cire de jojoba, et leurs mélanges.

3. Composition selon l'une des revendications précédentes, dans laquelle le motif est de formule (I) :
- avec R₁ = -isophorone- et R2 = méthyl, ce qui conduit au motif de formule : 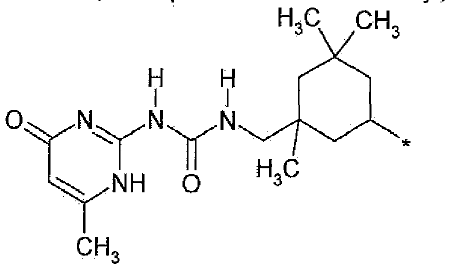
- avec R₁ = -(CH₂)₆-, R2 = méthyl, ce qui conduit au motif de formule : 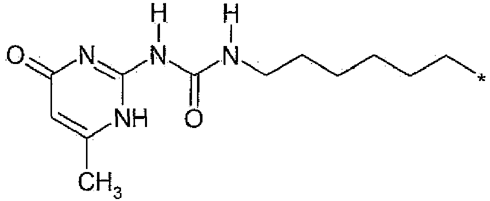
- avec R₁ = -(CH₂)₆-, R2 = isopropyl, ce qui conduit au motif de formule : 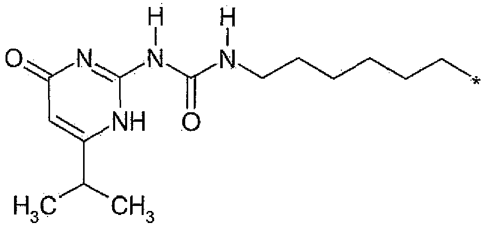 ou
- avec R₁ = 4,4'-méthylènebiseyclohexylène et R2 = méthyle, ce qui conduit au motif de formule :

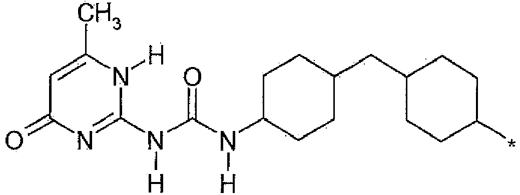

4. Composition selon l'une des revendications précédentes, dans laquelle le motif est de formule (II) avec R1= -isophorone-, R2= méthyle et R3=-(CH₂)₂OCO-NH-isophorone-, ce qui conduit au motif divalent de formule :

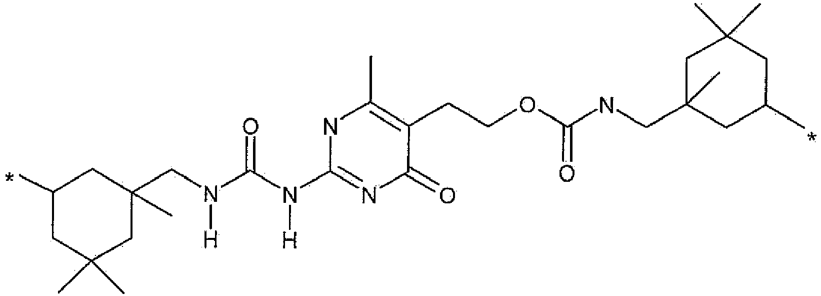

5. Composition selon l'une des revendications précédentes, dans laquelle le groupe de jonction est choisi parmi les groupes suivants :

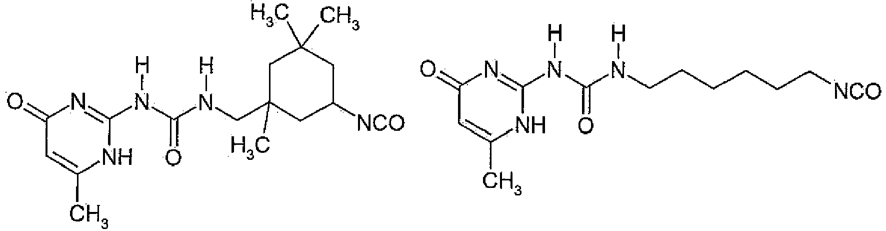

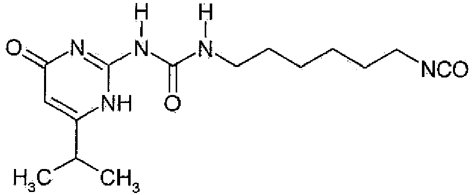

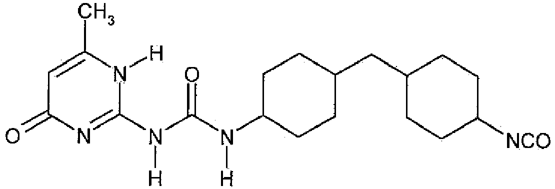

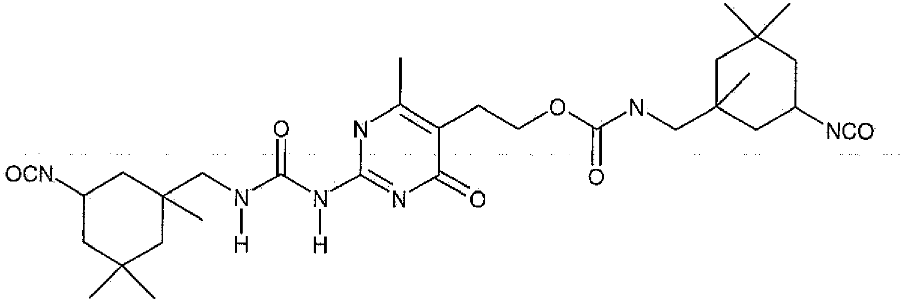

6. Composition selon l'une des revendications précédentes, dans laquelle le composé est présent en une quantité comprise entre 5 et 80% en poids, de préférence entre 10 et 75% en poids, notamment entre 20 et 70% en poids, voire entre 25 et 65% en poids, et mieux entre 30 et 60% en poids, par rapport au poids de la composition cosmétique finale.

7. Composition selon l'une des revendications précédentes, comprenant au moins un constituant choisi parmi les huiles et/ou solvants d'origine minérale, animale, végétale ou synthétique, carbonés, hydrocarbonés, fluorés et/ou siliconés; les agents épaississants; les cires d'origine végétale, animale, minérale ou de synthèse, voire siliconée; les matières colorantes; les antioxydants, les parfums, les huiles essentielles, les conservateurs, les actifs cosmétiques, les hydratants, les vitamines, les céramides, les filtres solaires, les tensioactifs, les gélifiants, les agents d'étalement, les agents mouillants, les agents dispersants, les anti-mousses, les neutralisants, les stabilisants, les polymères et notamment les polymères filmogènes liposolubles, et leurs mélanges.

8. Composition selon l'une des revendications précédentes, se présentant sous la forme d'un produit de soin et/ou de maquillage de la peau du corps ou du visage, des lèvres, des cils, des sourcils, des cheveux ou des ongles; d'un produit solaire ou autobronzant; d'un produit capillaire.

9. Procédé de traitement cosmétique des matières kératiniques, notamment de la peau du corps ou du visage, des lèvres, des ongles, des cils et/ou des cheveux, comprenant l'application sur lesdites matières d'une composition cosmétique telle que définie à l'une des revendications 1 à 8.

10. Composé susceptible d'être obtenu par réaction entre :
- au moins une cire portant au moins une fonction réactive choisie parmi OH, COOH, la cire étant choisie parmi la cire de Candellila, la cire de Carnauba, la cire de canne à sucre, la cire d'abeille, la cire de Montan, la cire de jojoba ;
et
- au moins un groupe de jonction capable d'établir des liaisons hydrogène avec un ou plusieurs groupes de jonction partenaires, chaque appariement d'un groupe de jonction faisant intervenir au moins 4 liaisons hydrogène, ledit groupe de jonction portant au moins une fonction réactive dite complémentaire, susceptible de réagir avec la fonction réactive portée par la cire, ledit groupe de jonction comprenant au moins un motif de formule (I) ou (II) : 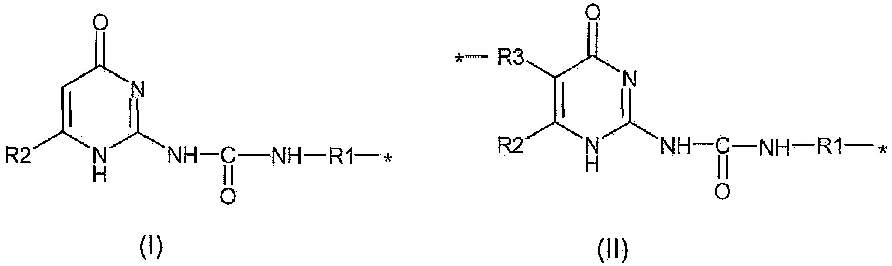 dans lesquelles :
- R1 et R3, identiques ou différents, représentent un radical carboné divalent choisi parmi (i) un groupe alkyle linéaire ou ramifié en C₁-C₃₂, (ii) un groupe cycloalkyle en C₄-C₁₆ et (iii) un groupe aryle en C₄-C₁₆; comprenant éventuellement 1 à 8 hétéroatomes choisis parmi O, N, S, F, Si et P; et/ou éventuellement substitué par une fonction ester, amide ou par un radical alkyle en C₁-C₁₂; ou un mélange de ces groupes;
- R2 représente un atome d'hydrogène ou un radical carboné, notamment hydrocarboné (alkyle), linéaire, ramifié ou cyclique, saturé ou insaturé, éventuellement aromatique, en C₁-C₃₂, pouvant comprendre un ou plusieurs hétéroatomes choisis parmi O, N, S, F, Si et P.

11. Composé selon la revendication 10, dans lequel la cire portant au moins une fonction réactive, notamment OH, COOH, est choisie parmi, seule ou en mélange :
la cire d'abeille, la cire de Carnauba et la cire de jojoba.

12. Composé selon la revendication 10 ou 11, dans lequel le groupe de jonction est choisi parmi les groupes suivants :

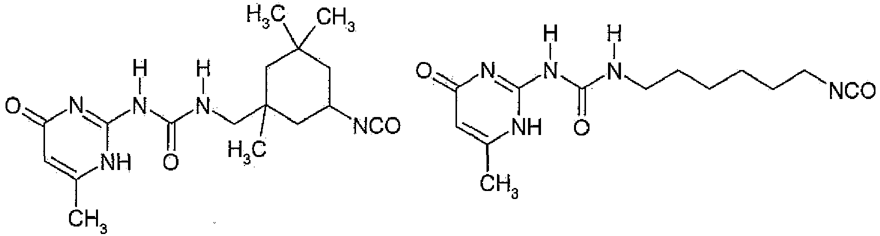

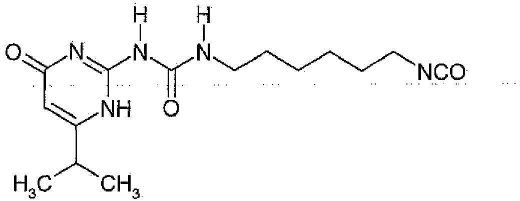

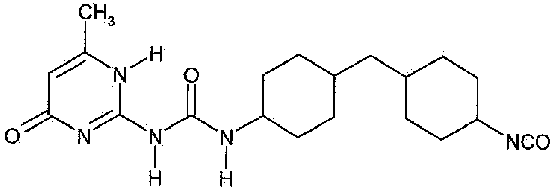

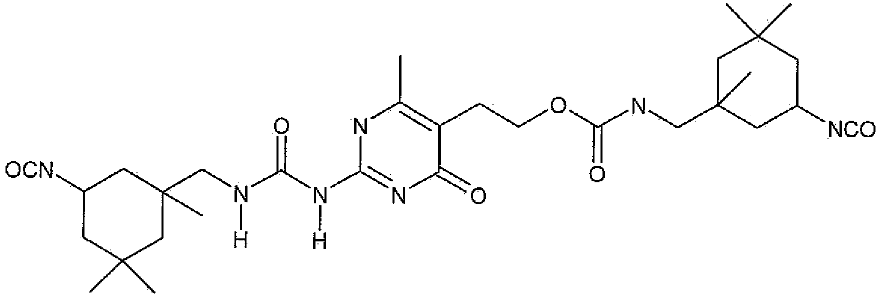

## Patentansprüche

1. Kosmetikzusammensetzung, die in einem kosmetisch unbedenklichen Medium eine Verbindung umfasst, die durch eine Reaktion zwischen den Folgenden erhältlich ist:
- mindestens einem Wachs, das mindestens eine reaktionsfähige Funktion ausgewählt aus OH oder COOH trägt, wobei das Wachs aus Candelillawachs, Carnaubawachs, Zuckerrohrwachs, Bienenwachs, Montanwachs und Jojobawachs ausgewählt ist; und
- mindestens einer Verbindungsgruppe, die fähig ist, Wasserstoffbrückenbindungen mit einer oder mehreren Partner-Verbindungsgruppen einzugehen, wobei an jeder Paarung einer Verbindungsgruppe mindestens vier Wasserstoffbrückenbindungen beteiligt sind, wobei die Verbindungsgruppe mindestens eine reaktionsfähige Funktion, die als komplementär bezeichnet wird, trägt, die mit der reaktionsfähigen Funktion, die von dem Wachs getragen wird, reagieren kann, wobei die Verbindungsgruppe mindestens eine Einheit der Formel (I) oder (II) umfasst: 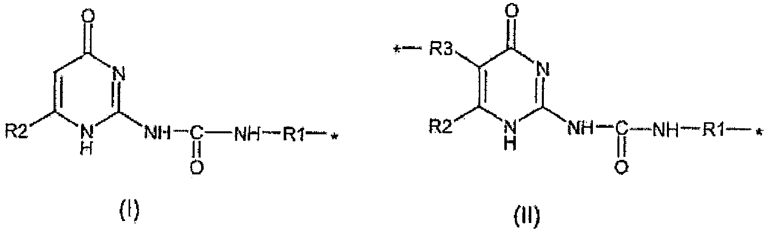 worin:
- R1 und R3, die gleich oder verschieden sind, für einen zweiwertigen Kohlenstoffrest stehen, der unter (i) einer linearen oder verzweigten C₁-C₃₂-Alkylgruppe, (ii) einer C₄-C₁₆-Cycloalkylgruppe und (iii) einer C₄-C₁₆-Arylgruppe ausgewählt ist, die gegebenenfalls 1 bis 8 unter O, N, S, F, Si und P ausgewählte Heteroatome umfasst und/oder gegebenenfalls durch eine Ester- oder Amidfunktion oder einen C₁-C₁₂-Alkylrest substituiert ist, oder eine Mischung dieser Gruppen bedeuten;
- R2 ein Wasserstoffatom oder einen linearen, verzweigten oder cyclischen, gesättigten oder ungesättigten, gegebenenfalls aromatischen C₁-C₃₂-Kohlenstoffrest, insbesondere Kohlenwasserstoffrest (Alkylrest), der ein oder mehrere unter 0, N, S, F, Si und P ausgewählte Heteroatome umfassen kann, bedeutet.

2. Zusammensetzung nach Anspruch 1, worin das Wachs, das mindestens eine reaktionsfähige Funktion, insbesondere OH, COOH, trägt, aus den Folgenden, allein oder als Mischung, ausgewählt ist:
Bienenwachs, Carnaubawachs und Jojobawachs sowie ihre Mischungen.

3. Zusammensetzung nach einem der vorhergehenden Ansprüche, worin die Einheit die Formel (I) aufweist:
- wobei R₁ = -Isophoron- und R2 = Methyl, was zu einer Einheit der Formel 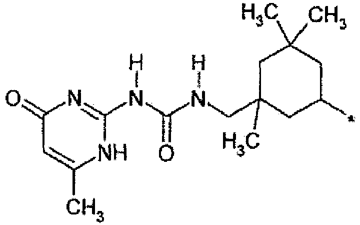 führt:
- wobei R₁ = - (CH₂)₆-, R2 = Methyl, was zur Einheit der Formel 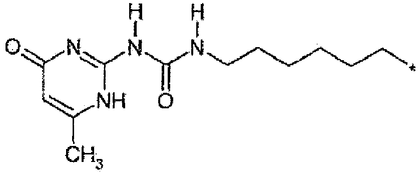 führt,
- wobei R₁ = -(CH₂)₆-, R2 = Isopropyl, was zur Einheit der Formel 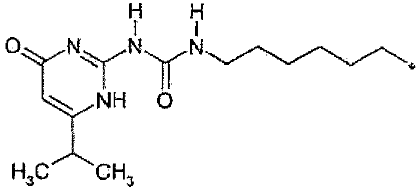 führt, oder
- wobei R₂ = 4,4'-Methylenbiscyclohexylen und R2 = Methyl, was zur Einheit der Formel 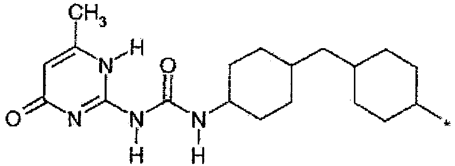 führt.

4. Zusammensetzung nach einem der vorhergehenden Ansprüche, worin die Einheit der Formel (II) entspricht, wobei R1 = -Isophoron-, R2 = Methyl und R3 = -(CH₂)₂OCO-NH-Isophoron-, was zu einer zweiwertigen Einheit der Formel: 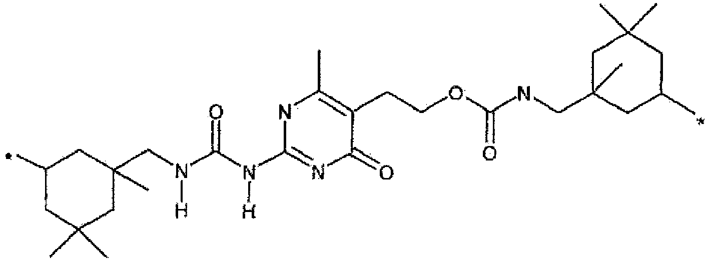 führt.

5. Zusammensetzung nach einem der vorhergehenden Ansprüche, worin die Verbindungsgruppe aus den folgenden Gruppen ausgewählt ist:

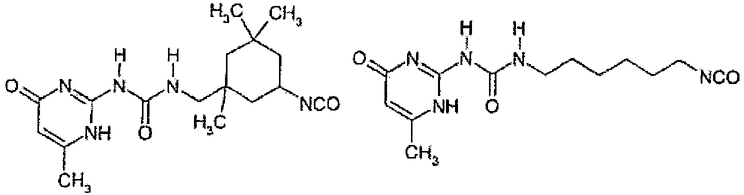

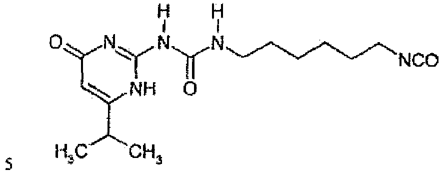

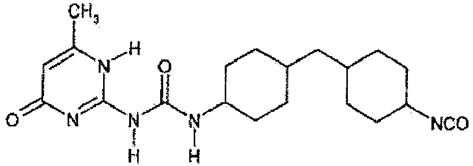

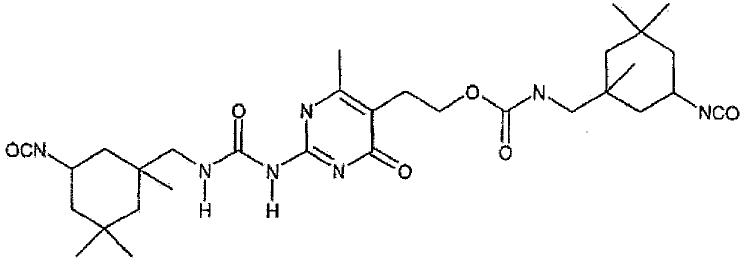

6. Zusammensetzung nach einem der vorhergehenden Ansprüche, worin die Zusammensetzung in einer Menge zwischen 5 und 80 Gew.-%, vorzugsweise zwischen 10 und 75 Gew.-%, insbesondere zwischen 20 und 70 Gew.-%, nämlich zwischen 25 und 65 Gew.-%, besser zwischen 30 und 60 Gew.-%, in Bezug auf das Gewicht der Endkosmetikzusammensetzung vorliegt.

7. Zusammensetzung nach einem der vorhergehenden Ansprüche, die mindestens einen Bestandteil, ausgewählt aus den Folgenden, umfasst: kohlenstoffhaltigen, kohlenwasserstoffhaltigen, fluorhaltigen und/oder silikönhaltigen Ölen und/oder Lösungsmitteln mineralischen, tierischen, pflanzlichen oder synthetischen Ursprungs; Verdickungsmitteln; Wachsen pflanzlichen, tierischen, mineralischen oder synthetischen Ursprungs, auch Silikonwachsen; Farbstoffen; Antioxidantien, Duftstoffen, etherischen Ölen, Konservierungsstoffen, kosmetischen Wirkstoffen, feuchtigkeitsspendenden Mitteln, Vitaminen, Ceramiden, Sonnenschutzfiltern, Tensiden, Gelbildnern, Spreitmitteln, Netzmitteln, Dispergiermitteln, Schaumhemmern, Neutralisierungsmitteln, Stabilisatoren, Polymeren und insbesondere lipidlöslichen filmbildenden Polymeren, und ihre Mischungen.

8. Zusammensetzung nach einem der vorhergehenden Ansprüche, die in Form eines Pflege- und/oder Make-up-Produkts für die Haut des Körpers oder des Gesichts, Lippen, Wimpern, Augenbrauen, das Haar oder Nägel; eines Sonnenschutzprodukts oder Selbstbräunungsmittels; eines Haarprodukts vorliegt.

9. Verfahren zur kosmetischen Behandlung von Keratinsubstanzen, insbesondere der Haut des Körpers oder des Gesichts, der Lippen, der Nägel, der Wimpern und/oder des Haars, bei dem man eine Kosmetikzusammensetzung wie in einem der Ansprüche 1 bis 8 definiert auf diese Substanzen aufbringt.

10. Verbindung, die durch Reaktion zwischen den Folgenden erhältlich ist:
- mindestens einem Wachs, das mindestens eine reaktionsfähige Funktion ausgewählt aus OH oder COOH trägt, wobei das Wachs aus Candelillawachs, Carnaubawachs, Zuckerrohrwachs, Bienenwachs, Montanwachs und Jojobawachs ausgewählt ist; und
- mindestens einer Verbindungsgruppe, die fähig ist, Wasserstoffbrückenbindungen mit einer oder mehreren Partner-Verbindungsgruppen einzugehen, wobei an jeder Paarung einer Verbindungsgruppe mindestens vier Wasserstoffbrückenbindungen beteiligt sind, wobei die Verbindungsgruppe mindestens eine reaktionsfähige Funktion, die als komplementär bezeichnet wird, trägt, die mit der reaktionsfähigen Funktion, die von dem Wachs getragen wird, reagieren kann, wobei die Verbindungsgruppe mindestens eine Einheit der Formel (I) oder (II) umfasst: 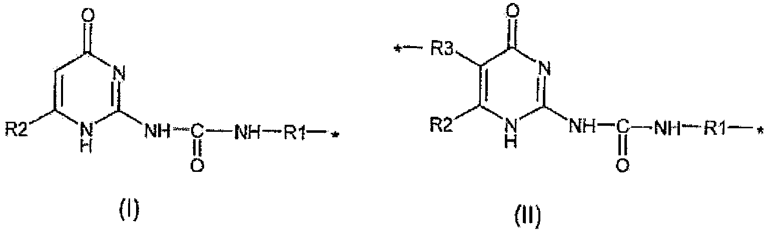 worin:
- R1 und R3, die gleich oder verschieden sind, für einen zweiwertigen Kohlenstoffrest stehen, der unter (i) einer linearen oder verzweigten C₁-C₃₂-Alkylgruppe, (ii) einer C₄-C₁₆-Cycloalkylgruppe und (iii) einer C₄-C₁₆-Arylgruppe ausgewählt ist, die gegebenenfalls 1 bis 8 unter O, N, S, F, Si und P ausgewählte Heteroatome umfasst und/oder gegebenenfalls durch eine Ester- oder Amidfunktion oder einen C₁-C₁₂-Alkylrest substituiert ist, oder eine Mischung dieser Gruppen bedeuten;
- R2 ein Wasserstoffatom oder einen linearen, verzweigten oder cyclischen, gesättigten oder ungesättigten, gegebenenfalls aromatischen C₁-C₃₂-Kohlenstoffrest, insbesondere Kohlenwasserstoffrest (Alkylrest), der ein oder mehrere unter 0, N, S, F, Si und P ausgewählte Heteroatome umfassen kann, bedeutet.

11. Verbindung nach Anspruch 10, worin das Wachs, das mindestens eine reaktionsfähige Funktion, insbesondere OH, COOH, trägt, aus den Folgenden, allein oder als Mischung, ausgewählt ist:
Bienenwachs, Carnaubawachs und Jojobawachs.

12. Verbindung nach Anspruch 10 oder 11, worin die verbindungsgruppe aus den folgenden Gruppen ausgewählt ist:

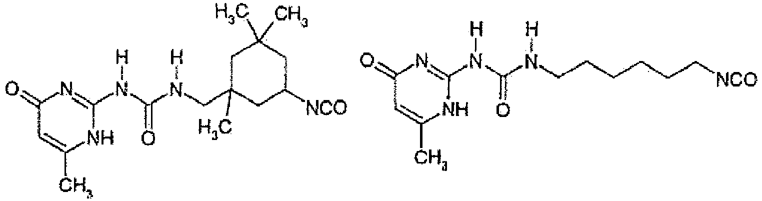

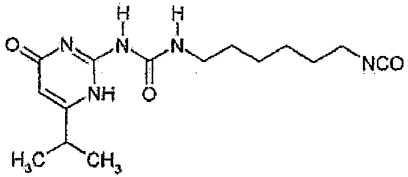

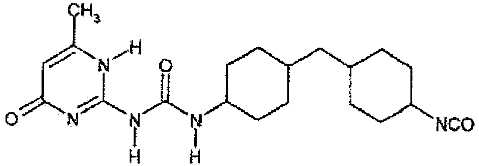

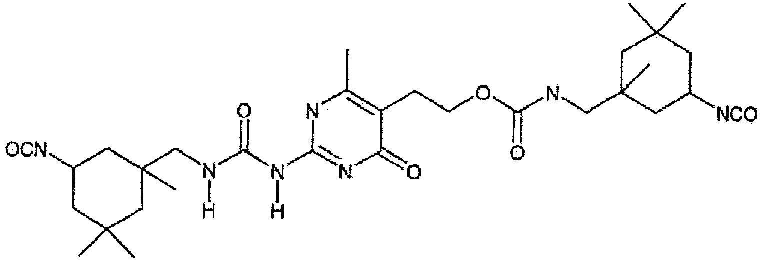

## Claims

1. Cosmetic composition comprising, in a cosmetically acceptable medium, a compound that can be obtained by reaction between:
- at least one wax bearing at least one reactive function chosen from OH or COOH, the wax being chosen from candelilla wax, carnauba wax, sugarcane wax, beeswax, montan wax and jojoba wax;
- at least one junction group capable of establishing hydrogen bonds with one or more partner junction groups, each pairing of a junction group involving at least 4 hydrogen bonds, said junction group bearing at least one "complementary" reactive function capable of reacting with the reactive function borne by the wax, said junction group comprising at least one unit of formula (I) or (II): 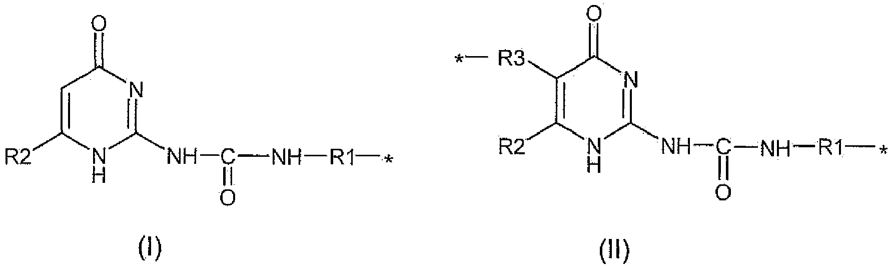 in which:
- R1 and R3, which may be identical or different, represent a divalent carbon-based radical chosen from (i) a linear or branched C₁-C₃₂ alkyl group, (ii) a C₄-C₁₆ cycloalkyl group and (iii) a C₄-C₁₆ aryl group; optionally comprising 1 to 8 heteroatoms chosen from O, N, S, F, Si and P; and/or optionally substituted with an ester or amide function or with a C₁-C₁₂ alkyl radical; or a mixture of these groups;
- R2 represents a hydrogen atom or a linear, branched or cyclic, saturated or unsaturated, optionally aromatic, C₁-C₃₂ carbon-based and especially hydrocarbon-based (alkyl) radical, which may comprise one or more heteroatoms chosen from O, N, S, F, Si and P.

2. Composition according to Claim 1, in which the wax bearing at least one reactive function, in particular OH or COOH, is chosen, alone or as a mixture, from: beeswax, carnauba wax and jojoba wax, and mixtures thereof.

3. Composition according to either of the preceding claims, in which the unit is of formula (I):
- with R1 = -isophorone-, R2 = methyl, which gives the unit of formula: 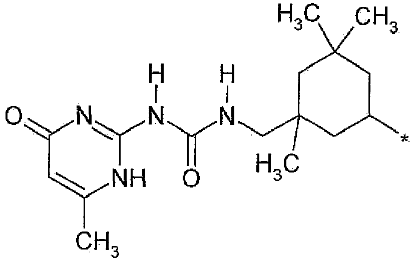
- with R1 = -(CH₂)₆-, R2 = methyl, which gives the unit of formula: 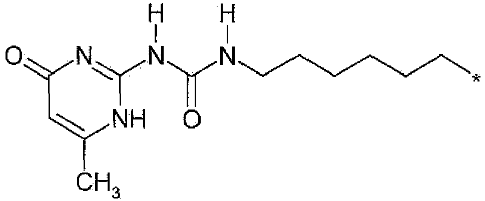
- with R1 = -(CH₂)₆-, R2 = isopropyl, which gives the unit of formula: 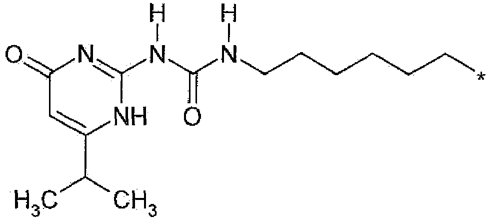 or
- with R1 = 4,4'-methylenebiscyclohexylene and R2 = methyl, which gives the unit of formula:

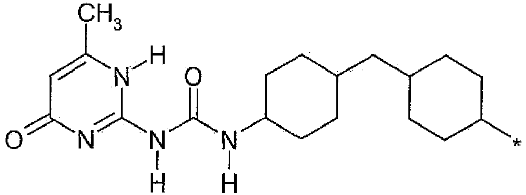

4. Composition according to one of the preceding claims, in which the unit is of formula (II) with R1 =-isophorone-, R2= methyl and R3=- (CH₂)₂OCO-NH-isophorone-which gives the unit of formula:

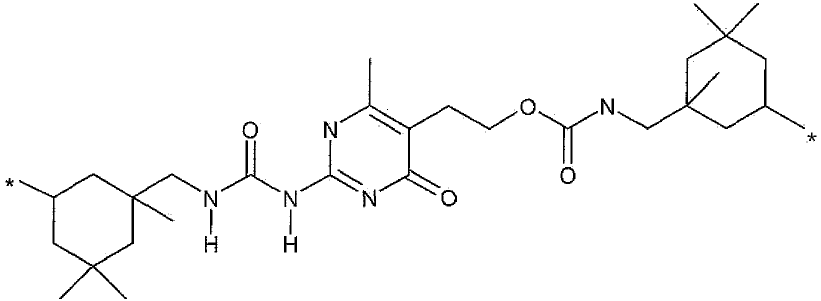

5. Composition according to one of the preceding claims, in which the junction group is chosen from the following groups:

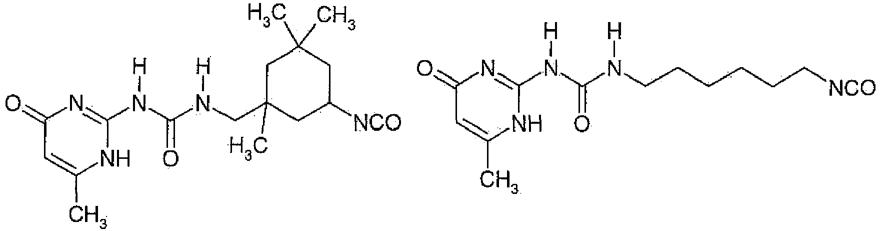

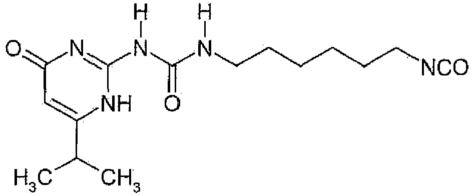

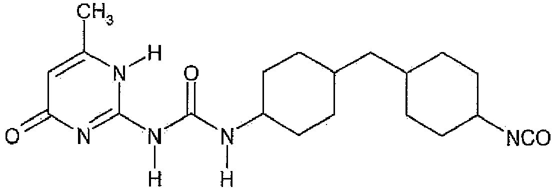

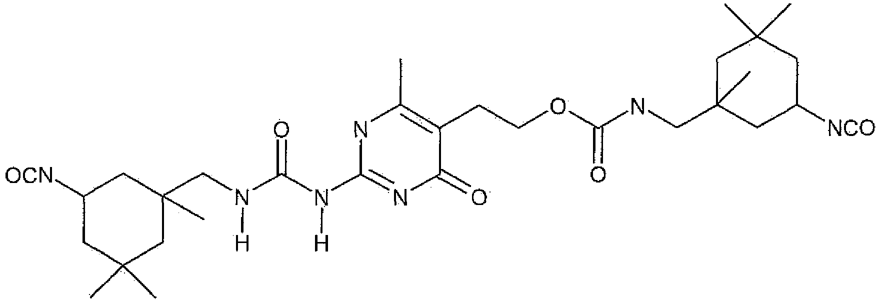

6. Composition according to one of the preceding claims, in which the compound is present in an amount of between 5% and 80% by weight, preferably between 10% and 75% by weight, in particular between 20% and 70% by weight, or even between 25% and 65% by weight, and better still between 30% and 60% by weight, relative to the weight of the final cosmetic composition.

7. Composition according to one of the preceding claims, comprising at least one constituent chosen from carbon-based, hydrocarbon-based, fluoro and/or silicone oils and/or solvents of mineral, animal, plant or synthetic origin;: thickeners; waxes of plant, animal, mineral or synthetic origin, or even silicone waxes; colorants; antioxidants, fragrances, essential oils, preservatives, cosmetic active agents, moisturizers, vitamins, ceramides, sunscreens, surfactants, gelling agents, spreading agents, wetting agents, dispersants, antifoams, neutralizing agents, stabilizers, polymers and in particular liposoluble film-forming polymers, and mixtures thereof.

8. Composition according to one of the preceding claims, which is in the form of a product for caring for and/or making up bodily or facial skin, the lips, the eyelashes, the eyebrows, the hair or the nails; an antisun or self-tanning product; or a hair product.

9. Cosmetic process for treating keratin materials, in particular bodily or facial skin, the lips, the nails, the eyelashes and/or the hair, comprising the application to said materials of a cosmetic composition as defined in one of Claims 1 to 8.

10. Compound that can be obtained by reaction between:
- at least one wax bearing at least one reactive function chosen from OH or COOH, the wax being chosen from candelilla wax, carnauba wax, sugarcane wax, beeswax, montan wax and jojoba wax; and
- at least one junction group capable of establishing hydrogen bonds with one or more partner junction groups, each pairing of a junction group involving at least 4 hydrogen bonds, said junction group bearing at least one "complementary" reactive function capable of reacting with the reactive function borne by the wax, said junction group comprising at least one unit of formula (I) or (II): 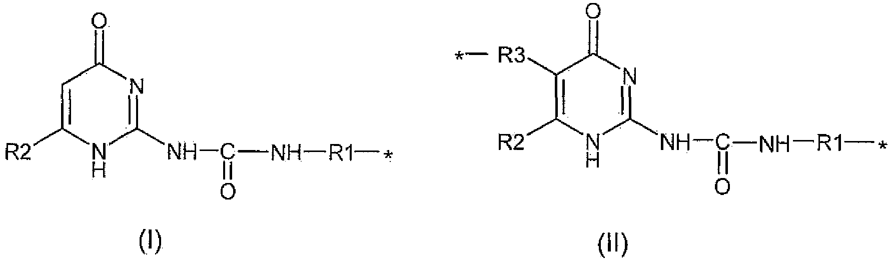 in which:
- R1 and R3, which may be identical or different, represent a divalent carbon-based radical chosen from (i) a linear or branched C₁-C₃₂ alkyl group, (ii) a C₄-C₁₆ cycloalkyl group and (iii) a C₄-C₁₆ aryl group; optionally comprising 1 to 8 heteroatoms chosen from O, N, S, F, Si and P; and/or optionally substituted with an ester or amide function or with a C₁-C₁₂ alkyl radical; or a mixture of these groups;
- R2 represents a hydrogen atom or a linear, branched or cyclic, saturated or unsaturated, optionally aromatic, C₁-C₃₂ carbon-based and in particular hydrocarbon-based (alkyl) radical, which may comprise one or more heteroatoms chosen from O, N, S, F, Si and P.

11. Compound according to Claim 10, in which the wax bearing at least one reactive function, in particular OH or COOH, is chosen, alone or as a mixture, from:
beeswax, carnauba wax and jojoba wax, and mixtures thereof.

12. Compound according to Claim. 10 or 11, in which the junction group is chosen from the following groups:
